# EUROPEAN PATENT SPECIFICATION

(11) **EP 1 978 365 B1**
(45) Date of publication and mention of the grant of the patent: **25.08.2010**
(21) Application number: 08152793.9
(22) Date of filing: 14.03.2008
(51) Int. Cl.: G01N 33/576

(54) **Reagent kit and method for measuring HCV antibody**
Reagenzkit und Verfahren zur Messung von HCV-Antikörpern
Kit de réactif et procédé de mesure d'anti-corps HCV

(30) Priority: 16.03.2007 JP 2007068810; 16.03.2007 JP 2007068818; 17.12.2007 JP 2007324927; 17.12.2007 JP 2007324919
(43) Date of publication of application: 08.10.2008
(73) Proprietor: SYSMEX CORPORATION, Hyogo 651-0073 (JP)
(72) Inventor: Masaki, Ichii, Hyogo 651-0073 (JP); Shunsuke, Matsubara, Hyogo 651-0073 (JP)
(74) Representative: De Clercq, Ann G. Y.

(56) References cited:
- EP-A- 1 365 240
- WO-A-02/04484
- US-B1- 6 613 530

## Description

### TECHNICAL FIELD

The present invention relates to a reagent kit and a method for measuring HCV antibody in a sample.

### BACKGROUND

Hepatitis C is an infectious disease caused by hepatitis C virus (HCV).

HCV is a virus having a single-stranded (+ chain) RNA genome of about 9.5 kb in full length, and its genome structure consists of noncoding and coding regions at the 5'-side and a noncoding region at the 3'-side.

In the coding region consisting of about 3000 amino acid residues, there are a region corresponding to structural proteins (a core protein and envelope proteins (E1, E2)) and a region corresponding to nonstructural proteins (NS2, NS3, NS4 and NS5).

As a method of detecting HCV, an immunological method is widely used. This method is carried out by detecting an antigen-antibody complex formed by binding an HCV antigen in a reagent to an HCV antibody in a sample. In immunological detection of an HCV antibody, HCV antigens having sequences corresponding respectively to various HCV proteins are used so as to enable detection of plural kinds of HCV antibodies recognizing different kinds of HCV proteins. HCV antigens can be isolated from the nature or produced by a genetic recombination method or a chemical synthesis method. Such HCV antigens include those disclosed in, for example, US Patent Nos. 5350671, 5910404 and 5308750.

A conventional reagent kit for immunologically detecting an HCV antibody contains a plurality of HCV antigens immobilized on a solid phase. For example, international patent application WO 02/04484 discloses the detection of anti-HCV antibodies using a highly immunoreactive mosaic antigen composition, which is immobilized on a solid support, comprising a plurality of antigenic peptides derived from the core region of a polyprotein encoded by the HCV genome.

In addition, US patent 6, 613, 530, discloses a method for detection of an antibody against HIV or HCV in a liquid sample. This method comprises incubating the liquid sample in the presence of a solid phase with two antigens against the antibody. The first antigen has at least one marker group and the second antigen binds to the solid phase under conditions to obtain an immune complex comprising a solid phase-bound second antigen to which the antibody is bound and to which the first antigen is bound. The antibody can be detected by direct or indirect detection of the marker group.

Another type of immunoassay is described in European patent application publication EP 1 365 240, wherein carrier particles carrying immobilized antigen are agglutinated when the substance to be detected (i.e. antibody) is present. Based on the measurement of the agglutination level of the carrier particles it is analyzed whether antibody is present in a sample or not.

Accordingly, in conventional immunodetection methods, the antigens are reacted with HCV antibodies in a sample to form antigen-antibody complexes on the solid phase, and these complexes are detected to measure the HCV antibodies.

### SUMMARY

In a process of studying a reagent for measuring an HCV antibody by using an HCV synthetic peptide antigen and an HCV recombinant antigen, the present inventors examined the stability and reactivity of reagents containing these antigens. As a result, they found that there is a difference in stability and reactivity between the HCV synthetic peptide antigen and the HCV recombinant antigen.

The reagent kit for measuring an HCV antibody according to the present invention comprises a first reagent and a second reagent. The first reagent contains an HCV synthetic peptide antigen having a solid phase binding site added thereto. The second reagent contains a solid phase on which both an HCV recombinant antigen and a binding substance capable of binding to the solid phase binding site were immobilized.

Alternatively, the second reagent may not be the second reagent described above and may be a second reagent containing a first solid phase having an HCV recombinant antigen immobilized thereon and a second solid phase on which a binding substance capable of binding to the solid phase binding site was immobilized.

A method for measuring an HCV antibody according to the present invention comprises contacting a sample, the first reagent, and the second reagent and then detecting a first complex and/or a second complex formed on the solid phase. This first complex contains the HCV synthetic peptide antigen and an HCV antibody contained in the sample. The second complex contains the HCV recombinant antigen and an HCV antibody contained in the sample.

In the present invention, the first reagent contains the HCV synthetic peptide antigen in a released state. In this specification, the "released state" means that the HCV synthetic peptide antigen is free in the reagent and not immobilized in any solid phases.

The reagent kit for measuring an HCV antibody according to the present invention enables stable measurement for a long time because the HCV antigens used in the measurement are excellent in storage stability and has excellent reactivity to an antibody. Use of the reagent kit for measuring an HCV antibody according to the present invention can prevent the reduction in measurement sensitivity caused by reduction in the storage stability of the HCV antigens, thus enabling measurement with higher accuracy.

### BRIEF DESCRIPTION OF THE DRAWINGS

Fig. 1 shows one example of the reagent kit wherein the first reagent is a solution and the second reagent is a suspension having a solid phase suspended in a buffer.
Fig. 2 shows the results of reference example 1.
Fig. 3 shows the results of positive serum 1 in reference example 2.
Fig. 4 shows the results of positive serum 2 in reference example 2.
Fig. 5 shows the results of measurement of the positive serum 7 in example 2.
Fig. 6 shows the results of measurement of the positive serum 8 in example 2.
Fig. 7 shows the results of measurement of the positive serum 9 in example 2.
Fig. 8 shows the results of measurement of the positive serum 10 in example 2.
Fig. 10 shows the results of measurement of the positive serum 12 in example 3.
Fig. 11 shows the results of measurement of the positive serum 13 in example 3.
Fig. 12 shows the results of measurement of the positive serum 14 in example 3.

### DESCRIPTION OF THE PREFERRED EMBODIMENTS

Hepatitis C (HCV) has a genome encoding a protein consisting of about 3000 amino acid residues. This protein consisting of about 3000 amino acid residues is composed of a structural protein domain (a core protein and envelope proteins (El and E2) ) and a nonstructural protein domain (NS2, NS3, NS4 and NS5), and each of these domains is known to be an antigen having at least one epitope. Examples of a protein encoded by the HCV genome and a sequence of each domain contained in the protein are described in, for example, US Patent No. 5350671.

Because HCV is a highly mutated virus, the sequence of a protein encoded by its genome cannot be definitely expressed. In this specification, the term "antigen" includes both a protein or peptide having antigenicity encoded by the HCV genome or its variants having antigenicity.

The reagent kit for measuring an HCV antibody in the present embodiment comprises a first reagent and a second reagent.

The first reagent comprises an HCV syntheticpeptide antigen, and the second reagent comprises an HCV recombinant peptide antigen. The HCV synthetic peptide antigen is free in the first reagent and the HCV recombinant peptide is immobilized on a solid phase. The HCV synthetic peptide has a solid phase binding site. This site is chemically added to the HCV synthetic peptide antigen. The second reagent comprises a binding substance as well as the HCV recombinant peptide antigen. The binding substance may be immobilized on the solid phase on which the HCV recombinant peptide antigen is immobilized, or may be immobilized on a different solid phase (secondsolidphase) from the solidphase (first solidphase) on which the HCV recombinant peptide antigen is immobilized. The binding substance is a compound which can be bind to the solid phase binding site added to the HCV synthetic peptide antigen.

Then, the principle of HCV measurement using the reagent kit for measuring an HCV antibody is described hereafter.

An HCV antibody contained in a sample can bind to the HCV synthetic peptide antigen in the first reagent and/or the HCV recombinant antigen in the second reagent. The solid phase binding site added to the HCV synthetic peptide antigen in the first reagent can bind to the binding substance in the second reagent. When a sample, the first reagent and the second reagent are contacted with one another, an HCV antibody in the sample binds to the HCV recombinant antigen, and an HCV antibody in the sample binds to the HCV synthetic peptide antigen. The HCV synthetic peptide antigen having the solid phase binding site added thereto can bind to the binding substance immobilized on the solid phase.

The HCV antibodies thus captured on the solid phase can be detected by a method known in the art.

The HCV synthetic peptide antigen is a peptide isolated and cleaved from the natural protein or synthesized by a chemical synthesis method and contains at least one epitope. Preferably, the HCV synthetic peptide antigen contains at least 8 consecutive amino acid residues of the HCV protein and contains 60 or less consecutive amino acid resides of the same protein. More preferably, the HCV synthetic peptide antigen contains at least 10 consecutive amino acid residues of the HCV protein and contains 50 or less consecutive amino acid residues of the same protein. The HCV synthetic peptide antigen is one having a molecular weight preferably in the range of 900 to 7500, more preferably in the range of 1000 to 6000.

The HCV synthetic peptide antigen can be obtained by chemical synthesis methods known in the art, such as a solid phase synthesis method, a fragment condensation method and a solution synthesis method. The synthetic peptide antigen is more preferably the one synthesized by the solid phase method described by Merrifield in J. Am. Chem. Soc., 85, p. 2149, 1963.

The HCV synthetic peptide antigen is preferably at least one member selected from the group consisting of a synthetic peptide NS4 antigen, a synthetic peptide NS5 antigen and a synthetic peptide core antigen. Two or more of these synthetic peptide antigens can be used. The sequences of these antigens are those described in, for example, US Patent No. 5350671, but are not limited thereto insofar as they have antigenicity, and their sequences may contain mutations. For example, the "synthetic peptide core antigen" when referred to in this specification means a synthetic peptide antigen having an amino acid sequence derived from the amino acid sequence of a core domain of HCV protein.

The HCV recombinant antigen is a peptide prepared by a genetic recombination method and contains at least one epitope. The HCV recombinant antigen has preferably at least 80 consecutive amino acid residues, more preferably at least 90 consecutive amino acid residues. The HCV recombinant antigen is one having a molecular weight of preferably 9000 or more, more preferably 10000 or more.

The HCV recombinant antigen can be prepared by a genetic recombination method known in the art. The HCV recombinant antigen can be prepared, for example, by constructing a DNA encoding an amino acid sequence of the desired antigen, then cloning this DNA in a suitable expression vector, and transforming a suitable host cell with the vector. The HCV recombinant antigen can be prepared by a genetic recombination method described in, for example, US Patent No. 5350671.

The HCV recombinant antigen is preferably at least one member selected from the group consisting of a recombinant NS3 antigen, a recombinant NS4 antigen, a recombinant NS5 antigen, and a recombinant core antigen. The sequences of these antigens are those described in, for example, US Patent No. 5350671, but are not limited thereto insofar as they have antigenicity, and their sequences may contain mutations. For example, the "recombinant NS3 antigen" when referred to in this specification means a recombinant antigen having an amino acid sequence of all or part of anNS3 domain of HCVprotein. This also applies to other antigens (NS4, NS5, the core antigen etc.).

A combination of the solid phase binding site and the binding substance is not particularly limited as long as they can be bound specifically to each other when contacted under the conditions where the reagent kit is used. Such combination is, for example, a combination of biotin and avidin or an avidin derivative, a hapten and an antihapten antibody, nickel and a histidine tag, or glutathione and glutathione-S-transferase. The combination of a hapten and an antihapten antibody includes, for example, a combination of dinitrophenol (DNP) and an anti-DNP antibody and is preferably a combination of biotin and avidin or an avidin derivative. More preferably, the solid phase binding site contains biotin and the binding substance is avidin or an avidin derivative. In this specification, the term "avidin or an avidin derivative" is intended to encompass avidin and streptavidin.

In this specification, "adding the solid phase binding site" to the HCV synthetic peptide antigen means that the HCV synthetic peptide antigen is reacted so as to have the solid phase binding site. This reaction may be suitably selected depending on the type of the solid phase binding site, and includes, for example, chemical bonding such as covalent bonding.

A method of adding the solid phase binding site to the HCV synthetic peptide antigen is known in the art. For example, when the solid phase binding site contains biotin, biotin can be bound to the antigen, for example, via a group having reactivity to an amino group, a thiol group or the like in the HCV synthetic peptide antigen. The group having reactivity to an amino group includes an NHS group, and the group having reactivity to a thiol group includes a maleimide group etc.

A method of immobilizing the binding substance onto the solid phase is known in the art. This immobilization can be carried out, for example, by a physical adsorption method, a covalent bonding method, an ionic bonding method, or a combination thereof.

When the binding substance is avidin or an avidin derivative, avidin or an avidin derivative can be immobilized directly onto the solid phase, for example, by physical adsorption. Alternatively, avidin or an avidin derivative can be immobilized onto the solid phase by binding avidin or an avidin derivative onto the solid phase to which a substance (for example, biotin) capable of binding to avidin or an avidin derivative was bound. Alternatively, avidin or an avidin derivative can be immobilized onto the solid phase by a method described in Japanese Patent Publication No. 2006-226689.

The solid phase to which avidin or an avidin derivative was bound can be purchased from, for example, JSR Corporation or Dynal Biotech.

A method of immobilizing the HCV recombinant antigen onto the solid phase is known in the art. This immobilization can be carried out, for example, by a physical adsorption method, a covalent bonding method, an ionic bonding method and a combination thereof.

Alternatively, the HCV recombinant antigen can be immobilized onto the solid phase via bonding between biotin and avidin or an avidin derivative. A combination of substances via which the HCV recombinant antigen is immobilized onto the solid phase include not only the combination of biotin and avidin or an avidin derivative as described above, but also a combination of a hapten and an antihapten antibody, nickel and a histidine tag, or glutathione and glutathione-S-transferase. The combination of a hapten and an antihapten antibody includes, for example, a combination of DNP and an anti-DNP antibody.

The means of immobilizing the HCV recombinant antigen onto the solid phase may be the same as, or different from, the means of immobilizing the binding substance onto the solid phase.

The first solid phase and the second solid phase may be produced from the same material or different materials, but are preferably produced from the same material from the viewpoint of ease in production of the reagent kit. The material is not particularly limited insofar as it is an usual solid-phase material used in immunoassay, and examples include latex, rubber, polyethylene, polypropylene, polystyrene, a styrene-butadiene copolymer, polyvinyl chloride, polyvinyl acetate, polyacrylamide, polymethacrylate, a styrene-methacrylate copolymer,polyglycidylmethacrylate,an acrolein-ethyleneglycol dimethacrylate copolymer, polyvinylidene difluoride (PVDF), polymer materials (silicone etc.), agarose, gelatin, red blood cells,silica gel,glass,inactive alumina,andinorganic materials (magnetic material etc.). These materials may be used alone or in combination.

The form of the solid phase is not particularly limited insofar as the solid phase is in the form of a usual solid phase used in immunoassay, for example, in the form of a microtiter plate, a test tube, beads, particles, and nanoparticles. The particles include magnetic particles, hydrophobic particles such as polystyrene latex, copolymer latex particles having hydrophilic groups such as an amino group and a carboxyl group on the surfaces of the particles, red blood cells and gelatin particles.

The solid phase is more preferably magnetic particles, latex particles or wells of microtiterplate.

The magnetic particles contain a base material which has magnetism. Such magnetic particles are known in the art, and the base material known in the art includes, for example, base materials using Fe₂O₃ and/or Fe₂O₄, cobalt, nickel, ferrite, magnetite etc. For the purpose of binding a protein or the like to the surface of a magnetic particle, the surface of the base material is more preferably coated with a polymer etc.

The microtiter plate is preferably composed of a material such as polystyrene and polypropylene, and a microtiter plate used in enzyme-linked immunosorbent assay (ELISA) can be usually preferably used.

As described above, the first reagent contains the HCV synthetic peptide antigen in a released state. The first reagent is more preferably in the form of a liquid in which the HCV synthetic peptide antigen is dissolved in a buffer solution not influencing the stability of the HCV synthetic peptide antigen and the reactivity thereof to an HCV antibody. Alternatively, the first reagent may be in the form of a solid obtained by lyophilization etc. of the HCV synthetic peptide antigen. In this case, this reagent is added to a suitable solvent such as water or the like at the time of measurement and used as a solution.

When the first reagent is in the form of a solution and contains plural kinds of HCV synthetic peptide antigens, the first reagent may contain the plural kinds of HCV synthetic peptide antigens in the same buffer solution or may contain a plurality of buffer solutions each containing one of the HCV synthetic peptide antigens. Preferably, the first reagent contains plural kinds of HCV synthetic peptide antigens in the same buffer solution.

When the first reagent is in the form of a solution, the concentration of the HCV synthetic peptide antigens can be suitably selected from the type and number of the HCV synthetic peptide antigens.

The buffer solution preferably has a pH value in the range of about 6. 5 to 8 . 0 and may be, for example, phosphate-buffered saline (PBS), a triethanolamine hydrochloride buffer (TEA), Tris-HCl buffer or the like . The buffer may contain known additives such as a surfactant, a preservative and serum proteins.

When the second reagent contains plural kinds of HCV recombinant antigens, the second reagent may contain a solid phase having the plural kinds of HCV recombinant antigens immobilized thereon or may contain plural kinds of solid phases each having one of the HCV recombinant antigens immobilized thereon, or may be a combination thereof.

The second reagent is provided preferably in the state in which the solid phase is suspended in, or contacted with, a suitable buffer. When the second reagent contains a first solid phase and a second solid phase, the first solid phase and the second solid phase may be suspended in, or contacted with, different buffers, but are preferably suspended in the same buffer from the viewpoint of ease in using and handling the reagent kit. The state in which the solid phase is suspended in a buffer includes, for example, the state in which particles such as magnetic particles and latex particles are suspended as the solid phase in a buffer. The state in which the solid phase is contacted with a buffer includes, for example, the state in which a buffer is added to a well of a microtiter plate as the solid phase.

When the second reagent contains a first solid phase and a second solid phase, the quantitative ratio of the first solid phase to the second solid phase can be suitably selected depending on the type and amount of the HCV synthetic peptide antigen in the first reagent, the type and amount of the HCV recombinant antigen immobilized on the first solid phase and the type and amount of the binding substance immobilized on the second solid phase. For example, when both the first solid phase and the second solid phase are magnetic particles, the number of magnetic particles contained in the second reagent may be established such that the magnetic particles are contained in the first solid phase : the second solid phase in the range of 1 to 10 : 10 to 1.

As the buffer described above, the same buffer as usable in the first reagent may be used.

When both the HCV recombinant antigen and the binding substance are immobilized on the solidphase contained in the second reagent, the HCV recombinant antigen in an amount sufficient for binding of an HCV antibody contained in a sample, and the binding substance in an amount sufficient for binding of the HCV synthetic peptide antigen contained in the first reagent, should be present on the solid phase. Their quantitative ratio can be suitably selected depending on the type and amount of the HCV synthetic peptide antigen in the first reagent, the type of the HCV recombinant antigen and the type of the binding substance. For example, the ratio of the number of HCV recombinant antigen molecules to the number of binding substance molecules immobilized on the solid phase may be selected such that the HCV recombinant antigen : the binding substance is in the range of 1 : 10 to 10 : 1. When the HCV recombinant antigen is a recombinant NS3 antigen and the binding substance is avidin or an avidin derivative, the recombinant NS3 antigen : avidin or an avidin derivative may be in the range of 1 _{:} 2 to 8.

Preferably the reagent kit for measuring an HCV antibody further contains a third reagent containing a substance capable of binding to an HCV antibody labeled with a labeling substance.

The substance capable of binding to an HCV antibody includes an antibody to an HCV antibody, an HCV antigen, etc. The antibody exemplified herein also includes antibody fragments and derivatives thereof. Specific examples include an Fab fragment, F (ab') fragment, F (ab)₂ fragment and sFv fragment. The antibody class includes, but is not limited to, IgG and IgM.

The labeling substance is not particularly limited insofar as it is a labeling substance that can be used in usual immunoassay, and examples include an enzyme, a fluorescent substance and a radioisotope. The enzyme includes, for example, alkali phosphatase, peroxidase, glucose oxidase, tyrosinase, acid phosphatase etc. The fluorescent substance includes fluorescein isothiocyanate (FITC), green fluorescence protein (GFP), luciferin etc. The radioisotope includes ¹²⁵I, ¹⁴C, ³²P etc.

When the labeling substance is an enzyme, the reagent kit for measuring an HCV antibody preferably further contains a fourth reagent containing a substrate for the enzyme.

As the substrate, a substrate for the enzyme, known in the art, can be used. When the enzyme used is alkali phosphatase, a luminescent substrate, a colorimetric substrate etc. known in the art may be used, and examples include chemiluminescent substrates such as CDP-star^{®} (4-chloro-3-(methoxyspiro{1,2-dioxetane-3,2'-(5'-chloro)tricyclo[3. 3.1.1^{3.7}] decane}-4-yl) disodiumphenylphosphate) and CSPD^{®} (3-(4-methoxyspiro{1,2-dioxetane-3,2-(5'-chloro) tricyclo[3. 3.1.1^{3.7}] decane}-4-yl) disodiumphenylphosphate) and colorimetric substrates such as p-nitrophenyl phosphate, 5-bromo-4-chloro-3-indolyl-phosphoric acid (BCIP), 4-nitro blue tetrazolium chloride (NBT), and iodotetrazolium (INT).

The fourth reagent is preferably in the form of a solution containing the substrate dissolved in a suitable buffer. The concentration of the substrate can be suitably selected depending on the type of the substrate. The buffer usable in the fourth reagent can be suitably selected depending on the type of the substrate, and buffers known in the art can be used.

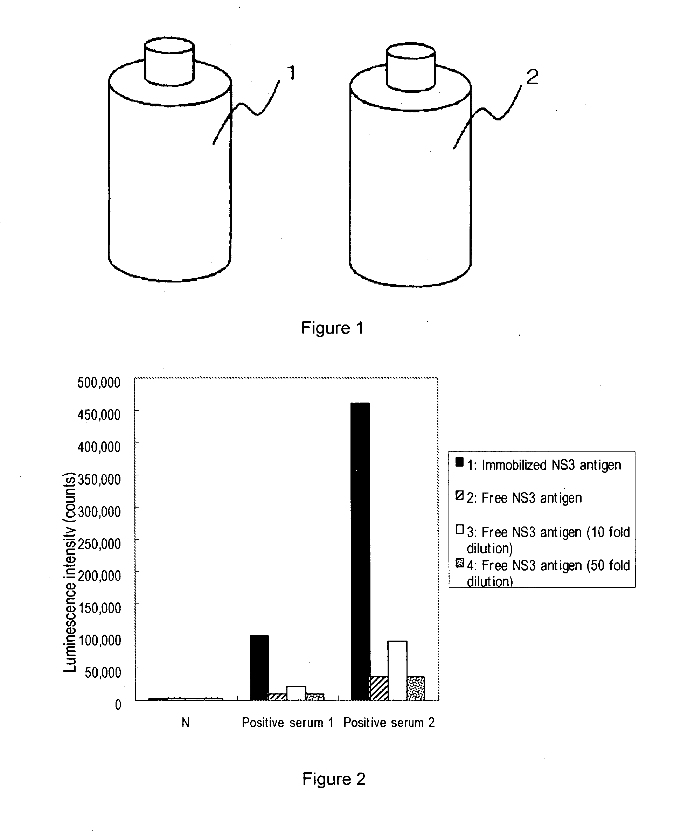

In the reagent kit for measuring an HCV antibody, the first reagent and the second reagent are packaged separately. Fig. 1 shows one example of the reagent kit wherein the first reagent is a solution and the second reagent is a suspension having a solid phase suspended in a buffer. In Fig. 1, the first reagent is accommodated in a first reagent container 1, and the second reagent is accommodated in a second reagent container 2. The reagent kit for measuring an HCV antibody maybe provided if desired with another container in which another reagent (for example, the third or fourth reagent described above) is accommodated. Further, the reagent kit for measuring an HCV antibody may be provided if desired with one or more buffers for diluting one or more reagents.

The method for measuring an HCV antibody in the present embodiment comprises contacting a sample, the first reagent, and the second reagent (contacting step), and
detecting a first complex of the HCV synthetic peptide antigen with an HCV antibody and/or a second complex of the HCV recombinant antigen and an HCV antibody (detection step).

The sample is a biological sample collected from an animal and includes, for example, blood, serum and plasma.

In the contacting step, a sample may be contacted first with the first reagent and then with the second reagent, or a sample may be contacted first with the second reagent and then with the first reagent. Alternatively, the first reagent and the second reagent may be first contacted with each other and then with a sample. Preferably, a sample is contacted first with the first reagent and then with the second reagent because the bonding between a free HCV antibody in the sample and the free HCV antigen in the reagentcaneasilyproceed. The term "contact" includes mixing a sample, the first reagent, and the second reagent.

The contacting step is carried out preferably at the temperature at which the reaction between an HCV antibody in a sample and the HCV antigens in the first and second reagents proceeds well, more preferably at a temperature of about 30 to 45°.

The contacting time may be suitably selected depending on the type etc. of the HCV synthetic peptide antigen, the HCV recombinant antigen, the solid phase binding site and the binding substance used.

The detection step may be carried out according to an immunological detection method known in the art. The detection step is carried out preferably by using a substance capable of binding to an HCV antibody labeled with the labeling substance.

The detection step is carried out preferably by contacting the substance capable of binding to an HCV antibody labeled with the labeling substance, with the first complex and/or the second complex, and then detecting the labeling substance.

When the labeling substance is an enzyme, the labeling substance can be detected by measuring, with a suitable apparatus, a light, a color or the like generated by reacting a substrate for the enzyme substrate with the enzyme. The apparatus includes a spectrophotometer, a luminometer etc.

### EXPERIMENTS

The method of preparing each of HCV antigens used in the Examples is described.

### (1) Preparation of Recombinant NS3 Antigen

As the HCV recombinant antigen, a recombinant NS3 antigen having an amino acid sequence of from amino acid 1192 to 1457 in the amino acid sequence shown in Fig. 1 in Japanese Patent Publication No. H05-508219 was prepared as described in Example 1 in Japanese Patent Publication No. H05-508219.

### (2) Preparation of Synthetic Peptide NS4 Antigen

As the synthetic peptide antigen, a synthetic peptide NS4 antigen having an amino acid sequence of from amino acid 1688 to 1707 (peptide VIII in Japanese Patent No. 2995216) in the amino acid sequence shown in Fig. 1 in Japanese Patent No. 2995216 was prepared according to a peptide synthesis method described in the Examples of Japanese Patent No. 2995216. Then, the synthetic peptide NS4 antigen was biotinylated with a biotinylation kit (Sulfo-OSu) (DOJINDO LABORATORIES).

### (3) Preparation of Synthetic Peptide Core Antigen

As the synthetic peptide antigens, a synthetic peptide core antigen having an amino acid sequence of from amino acid 7 to 26 (peptide II in Japanese Patent No. 2995216), a synthetic peptide core antigen having an amino acid sequence of amino acid 13 to 32 (peptide III in Japanese Patent No. 2995216) and a synthetic peptide core antigen having an amino acid sequence of amino acid 49 to 68 (peptide V in Japanese PatentNo. 2995216), in the amino acid sequence shown in Fig. 1 in Japanese Patent No. 2995216, were prepared according to a peptide synthesis method described in the Examples in Japanese Patent No. 2995216. Then, these synthetic peptide core antigens were biotinylated with a biotinylation kit (Sulfo-OSu) (DOJINDO LABORATORIES).

### (4) Preparation of Recombinant NS3 Antigen-Immobilized Magnetic Particles

Commercially available magnetic particles (average particle size 2 µm) were suspended at a density of about 5 mg/mL in 20 mM sodium phosphate buffer, pH 7.5, to prepare a magnetic particle suspension. The recombinant NS3 antigen prepared in (1) above was dissolved at a concentration of 1 mg/mL in 20 mM sodium phosphate buffer, pH 7.5, to prepare an NS3 antigen solution. 0.1 mL of the NS3 antigen solution was mixed with 10 mL of the magnetic particle suspension and incubated at 37°C for 30 minutes, whereby the recombinant NS3 antigen was immobilized onto the magnetic particles. Then, the magnetic particles having the NS3 antigen immobilized thereon were blocked by mixing the magnetic particles with a blocking solution (1% BSA, 20 mM sodium phosphate buffer, pH 7.5) and then incubating the mixture at 37°C for 30 minutes. The magnetic particles thus obtained are hereinafter referred to as the NS3 antigen magnetic particles.

### (5) Preparation of Synthetic Peptide NS4 Antigen-Immobilized Magnetic Particles

Commercially available streptavidin-coated magnetic particles (referred to hereinafter as "ST magnetic particles") were suspended at a density of about 5 mg/mL in 20 mM sodiumphosphate buffer, pH 7.5, to prepare an ST magnetic particle suspension. The synthetic peptide NS4 antigen prepared in (2) above was dissolved at a concentration of 1 mg/mL in 20 mM sodium phosphate buffer, pH 7.5, to prepare an NS4 antigen solution. 0.01 mL of the NS4 antigen solution was mixed with 10 mL of the ST magnetic particle suspension and incubated at 37°C for 30 minutes, whereby the synthetic peptide NS4 antigen was immobilized onto the ST magnetic particles. Then, the magnetic particles having the NS4 antigen immobilized thereon were blocked by mixing the magnetic particles with a blocking solution (1% BSA, 20 mM sodium phosphate buffer, pH 7.5) and then incubating the mixture at 37°C for 30 minutes. The magnetic particles thus obtained are hereinafter referred to as the NS4 antigen magnetic particles.

### (6) Preparation of Synthetic Peptide Core Antigen-Immobilized Magnetic Particles

Commercially available ST magnetic particles were suspended at a density of about 1 mg/mL in 20 mM sodium phosphate buffer, pH 7.5, to prepare an ST magnetic particle suspension. The synthetic peptide core antigens prepared in (3) above were mixed with one another and dissolved in 20 mM sodium phosphate buffer, pH 7.5, to prepare a core antigen solution. The core antigen solution was prepared such that each of the synthetic peptide core antigens contained therein was about 1 mg/mL. 0.01 mL of the core antigen solution was mixed with 10 mL of the ST magnetic particle suspension and incubated at 37°C for 30 minutes, whereby the synthetic peptide core antigens were immobilized onto the ST magnetic particles. Then, the magnetic particles having the core antigens immobilized thereon were blocked by mixing the magnetic particles with a blocking solution (1% BSA, 20 mM sodium phosphate buffer, pH 7.5) and then incubating the mixture at 37°C for 30 minutes. The magnetic particles thus obtained are hereinafter referred to as the core antigen magnetic particles.

### Reference Example 1

First, the difference between the reactivity of the recombinant NS3 antigen immobilized on magnetic particles and the reactivity of the same antigen which is free, without immobilization, in a solution was examined.

The biotinylated recombinant NS3 antigen obtained by biotinylating the NS3 antigen, prepared in (1) above, was used in this experiment. For biotinylating the NS3 antigen, a biotinylation kit (Sulfo-OSu) (DOJINDO LABORATORIES) was used.

The method of immobilizing the biotinylated NS3 antigen onto ST magnetic particles is the same as in (4) above except that the biotinylatedNS3 antigen was used as the HCV antigen, and ST magnetic particles were used as the magnetic particles.

The composition of each reagent used in this example is as follows:

### (7) First Reagent (A)

20 mM sodium phosphate buffer, pH 7.5

### (8) First Reagent (B-1)

20 mM sodium phosphate buffer, pH 7.5

2.5 µg/mL biotinylated NS3 antigen

### (9) First Reagent (B-2)

The first reagent (B-2) is prepared by diluting the first reagent (B-1) 10-fold with 20 mM sodium phosphate buffer, pH 7.5.

### (10) First Reagent (B-3)

The first reagent (B-3) is prepared by diluting the first reagent (B-1) 50-fold with 20 mM sodium phosphate buffer, pH 7.5.

### (11) Second Reagent (A)

20 mM sodium phosphate buffer, pH 7.5

3 mg/mL biotinylated NS3antigen-boundSTmagnetic particles

2 mg/mL ST magnetic particles

### (12) Second Reagent (B)

20 mM sodium phosphate buffer, pH 7.5

5 mg/mL ST magnetic particles

### (13) Third Reagent

20 mM sodium phosphate buffer, pH 7.5

0.1 U/mL alkali phosphatase-labeled mouse anti-human IgG antibody (ALKALINE PHOSPHATASE-MOUSE, Zymed Laboratories)

### (14) Fourth Reagent

CDP-star^{®} with Sapphire II^{®} (Applied Biosystems)

The measurement methods are as follows:

### (15) Reactivity of Recombinant NS3 Antigen When Immobilized on Magnetic Particles

First, 10 µL HCV-positive serum was mixed with 120 µL of the first reagent (A) and incubated at 42°C for about 2 minutes. Subsequently, 30 µL of the second reagent (A) was added to this mixture and incubated at 45°C for about 1 minute, thereby reacting an HCV antibody in the serum with the NS3 antigen on the magnetic particles in the second reagent (A) . The magnetic particles were washed with a wash (0.1% Tween 20, 20 mM sodium phosphate buffer, pH 7.5), and then 100 µL of the third reagent was added thereto and incubated at room temperature for 5 minutes. The magnetic particles were washed with the above wash, and then 100 µL of the fourth reagent was added to the mixture, to measure luminescence intensity with LUMI-COUNTER 700 (Microtec Co., Ltd.).

As the sample, two HCV-positive serums (positive serum 1 and positive serum 2) were used. As a negative control, 20 mM sodiumphosphate buffer, pH 7 . 5 was used in place of the HCV-positive serum and measured. The same sample was measured in triplicate to determine the average luminescence intensity. The results are shown in Fig. 2.

### (16) Reactivity of Recombinant NS3 Antigen Released into Solution

The measurement was carried out in the same manner as in (15) above except that the first reagent (B-1), (B-2) or (B-3) was used as the first reagent, and the second reagent (B) was used as the second reagent. The first reagent (B-1) contains the free NS3 antigen at the same concentration as that of the immobilized NS3 antigen in the second reagent A. The results are shown in Fig. 2.

In Fig. 2, "1" shows the results of the recombinant NS3 antigen immobilized on magnetic particles, and "2" to "4" show the results of the recombinant NS3 antigen released into a solution. The luminescence intensity determined by the measurement is shown on the ordinate. N is the results of the negative control.

From Fig. 2, it was found that in any HCV-positive serums, the recombinant NS3 antigen immobilized on magnetic particles give higher luminescence intensity than that of the recombinant NS3 antigen released into a solution. From the foregoing, it was found that the HCV recombinant antigen when immobilized on magnetic particles gives higher reactivity to an HCV antibody than when released into a solution.

### Reference Example 2

Then, the difference between the storage stability of the recombinant NS3 antigen immobilized on magnetic particles and the storage stability of the same antigen released, without immobilization, into a solution was examined.

In this example, the second reagent (A) (containing the immobilized NS3 antigen) and the first reagent (B-2) (containing the free NS3 antigen), used in Reference Example 1, were used in an acceleration test to examine the storage stability of the reagents. In the acceleration test, the second reagent (A) and the first reagent (B-2) were stored in the dark at 37°C for 0 day, 1 day or 5 days. Each reagent subjected to the acceleration test was used to measure an HCV antibody.

The acceleration test is a test wherein a reagent is examined for its storage stabilityetc. after storage at a temperature higher than the temperature at which the reagent should originally be stored. When storage stability is examined, the storage stability of a reagent is easily lost at a higher storage temperature, and thus the reagent is stored intentionally at a higher temperature thereby accelerating deterioration in the reagent. The storage stability can thereby be lost in a shorter time, and therefore, experimental results of the storage-stabilizing effect can be obtained in a shorter time.

The measurement methods are as follows:

### (17) Stability of Recombinant NS3 Antigen When Immobilized on Magnetic Particles

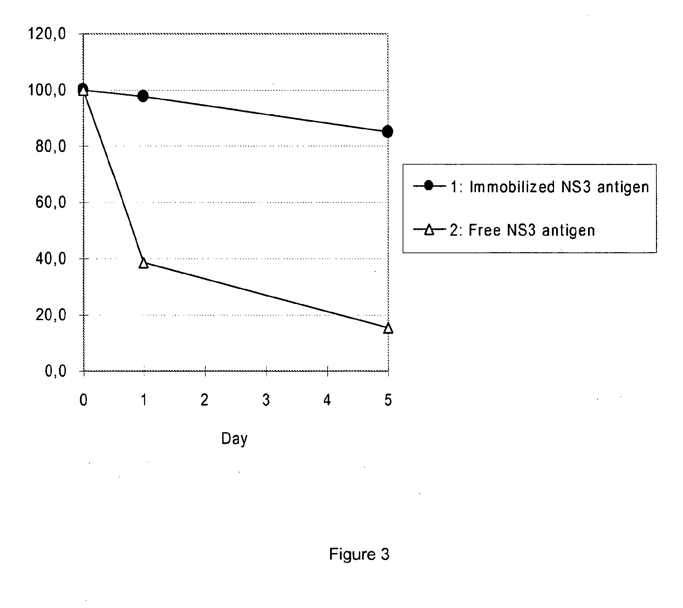

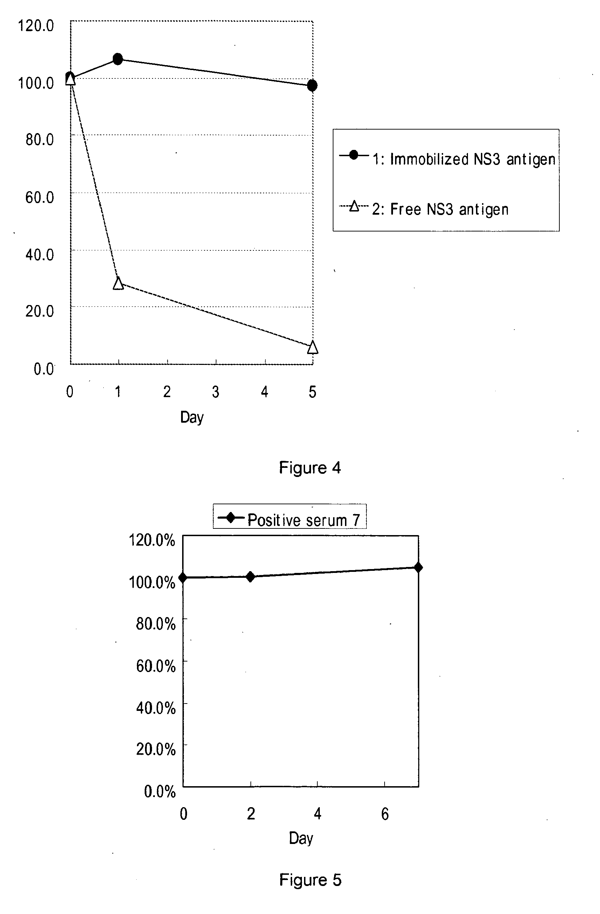

The measurement was carried out in the same manner as in (15) in Reference Example 1 except that the second reagent (A) subjected to the acceleration test was used as the second reagent. As the sample, the two HCV-positive serums (positive serum 1 and positive serum 2) used in Reference Example 1 were used. The results are shown in Figs. 3 and 4.

### (18) Stability of Recombinant NS3 Antigen which is free in a Solution

The measurement was carried out in the same manner as in (16) in Reference Example 1 except that the first reagent (B-2) subjected to the acceleration test was used as the first reagent. As the sample, the two HCV-positive serums (positive serum 1 and positive serum 2) used in Reference Example 1 were used. The results are shown in Figs. 3 and 4.

Fig. 3 shows the results of the antigen for positive serum 1 as the sample, and Fig. 4 shows the results of the antigen for positive serum 2 as the sample. In Figs. 3 and 4, "1" shows the results of the recombinant NS3 antigen immobilized on magnetic particles, and "2" shows the results of the recombinant NS3 antigen released into a solution. The percentage of average luminescence intensity on each day is shown on the ordinate where the average luminescence intensity obtained using the reagents before the acceleration test (day 0) is 100%. The number of days for which the reagents were stored is shown on the abscissa.

From the results in Figs. 3 and 4, it was found that the recombinant NS3 antigen, when stored in a state released into a solution, is poor in stability and reduces reactivity rapidly even after 1 day of the acceleration test. It was also found that the recombinant NS3 antigen, when stored in a state immobilized on magnetic particles, is excellent in stability and maintains high reactivity even after 5 days of the acceleration test. From the foregoing, it was found that the HCV recombinant antigen has higher storage stability when immobilized on magnetic particles than when released into a solution.

### Example 1

Then, the difference between the storage stability of the synthetic peptide NS4 antigen and the synthetic peptide core antigen immobilized on magnetic particles and the storage stability of the same antigens released, without immobilization, into a solution was examined.

In this example, the synthetic peptide NS4 antigen prepared in (2) above and the synthetic peptide core antigen prepared in (3) above were used as the released HCV antigen. The NS3 antigen magnetic particles prepared in (4) above, the NS4 antigen magnetic particles prepared in (5) above, and the core antigen magnetic particles prepared in (6) above were used as the immobilized HCV antigen.

The composition of each reagent used in this example is as follows. The third reagent and fourth reagent were the same as used in Reference Example 1.

### (19) First Reagent (C)

20 mM sodium phosphate buffer, pH 7.5

1 µg/mL synthetic peptide NS4 antigen

10 µg/mL each synthetic peptide core antigen

### (20) First Reagent (D)

20 mM sodium phosphate buffer, pH 7.5

### (21) Second Reagent (C)

20 mM sodium phosphate buffer, pH 7.5

3 mg/mL NS3 antigen magnetic particles

2 mg/mL ST magnetic particles

### (22) Second Reagent (D)

20 mM sodium phosphate buffer, pH 7.5

3 mg/mL NS3 antigen magnetic particles

1 mg/mL NS4 antigen magnetic particles

1 mg/mL core antigen magnetic particles

In this example, the first reagent (C) (containing the free NS4 antigen and the free core antigen) and the second reagent (D) (containing the immobilized NS4 antigen and the immobilized core antigen) were used in the acceleration test to examine the storage stability of the reagents. In the acceleration test, the first reagent (C) and the second reagent (D) were stored in the dark at 45°C for 0 day or 1 day. Each reagent subjected to the acceleration test was used to measure an HCV antibody.

The measurement methods are as follows:

### (23) Stability of Synthetic Peptide NS4 Antigen and Synthetic Peptide Core Antigen When Released into Solution

The measurement was carried out in the same manner as in (15) in Reference Example 1 except that the first reagent (C) subjected to the acceleration test was used as the first reagent, and the second reagent (C) was used as the second reagent. As the sample, one HCV-negative serum (negativeseruml)andfour HCV-positive serums (positive serums 3 to 6) were used. The results are shown in Table 1.

### (24) Stability of Synthetic Peptide NS4 Antigen and Synthetic Peptide Core Antigen When Immobilized on Magnetic Particles

The measurement was carried out in the same manner as in (15) in Reference Example 1 except that the first reagent (D) was used as the first reagent, and the second reagent (D) subjected to the acceleration test was used as the second reagent. As the sample, one HCV-negative serum (negative serum 1) and four HCV-positive serums (positive serums 3 to 6) were used. The results are shown in Table 1.

**Table 1**

| sample | Antigen immobilized on magnetic particles | | | Antigen free in solution | | |
|---|---|---|---|---|---|---|
| | Day 0 (Average luminescence intensity counts) | Day 1 (Average luminescence intensity: counts) | % | Day 0 (Average luminescence intensity: counts) | Day 1 (Average luminescence intensity counts) | % |
| Negative Serum 1 | 145,758 | 164,443 | 113 | 146,195 | 152,152 | 104 |
| Positive Serum 3 | 14,553,440 | 6,868,120 | 47 | 14,019,360 | 13,632,400 | 97 |
| Positive Serum 4 | 18,628,160 | 8,664,840 | 47 | 11,882,280 | 11,375,040 | 96 |
| Positive Serum 5 | 10,882,240 | 4,984,280 | 46 | 7,345,000 | 7,980,040 | 109 |
| Positive Serum 6 | 17,358,080 | 8,156,400 | 47 | 12,481,880 | 13,012,440 | 104 |

In Table 1, the average luminescence intensityobtainedusing the reagents before the acceleration test (day 0) and on day 1 of the acceleration test is shown. The percentage of average luminescence intensity obtained using the reagents on day 1 is expressed relative to the average luminescence intensity (that is, 100%) obtained using the same reagents on day 0.

From Table 1, it was found that the synthetic peptide NS4 antigen and the synthetic peptide core antigen, when stored in a state immobilized in magnetic particles, are poor in stability and rapidly reduce reactivity to all positive serums (positive serums 3 to 6), even after 1 day of the acceleration test. It was also found that the synthetic peptide NS4 antigen and the synthetic peptide core antigen, when stored in a state released to a solution, are excellent in stability and maintains high reactivity to all positive serums, even after 5 days of the acceleration test. From the foregoing, it was found that the HCV synthetic peptide antigen has higher storage stability when released into a solution than when immobilized in a solid phase.

### Example 2

The first reagent containing the synthetic peptide NS4 antigen and the synthetic peptide core antigen in a released form, and the second reagent containing the recombinant NS3 antigen in a state immobilized on magnetic particles, were used in the acceleration test to examine the storage stability of the reagents. In the acceleration test, the first reagent and the second reagent were stored in the dark at 37°C for 0 day, 2 days or 7 days. Each reagent subjected to the acceleration test was used to measure an HCV antibody. In this example, the first reagent (C) used in Example 1 was used as the first reagent, and the second reagent (C) used in Example was used as the second reagent.

The measurement method is as follows:

The measurement was carried out in the same manner as in (15) in Reference Example 1 except that the first reagent (C) subjected to the acceleration test was used as the first reagent, and the second reagent (C) subjected to the acceleration test was usedasthesecondreagent. As the sample, four HCV-positive serums (positive serums 7 to 10) were used. The positive serum 7 is a serum containing an HCV antibody to the NS3 antigen, an HCV antibody to the NS4 antigen and an HCV antibody to the core antigen. The positive serum 8 is a serum containing an HCV antibody to the NS3 antigen; the positive serum 9 is a serum containing an HCV antibody to the core antigen; and the positive serum 10 is a serum containing an HCV antibody to the NS3 antigen and an HCV antibody to the NS4 antigen. As the negative control, 20 mM sodium phosphate buffer, pH 7.5 was used in place of the HCV-positive serum and measured. The results are shown in Table 2.

**Table 2**

| sample | Preservation term | | |
|---|---|---|---|
| | Day 0 | Day 2 | Day 7 |
| | Average luminescence intensity (counts) | Average luminescence intensity (counts) | Average luminescence intensity (counts) |
| Negative control | 1.709 | 1.719 | 1.716 |
| Positive serum 7 | 274.452 | 275.827 | 288.173 |
| Positive serum 8 | 552.946 | 597.332 | 584.926 |
| Positive serum 9 | 361.389 | 397.041 | 385.178 |
| Positive serum 10 | 317.550 | 342.502 | 321.329 |

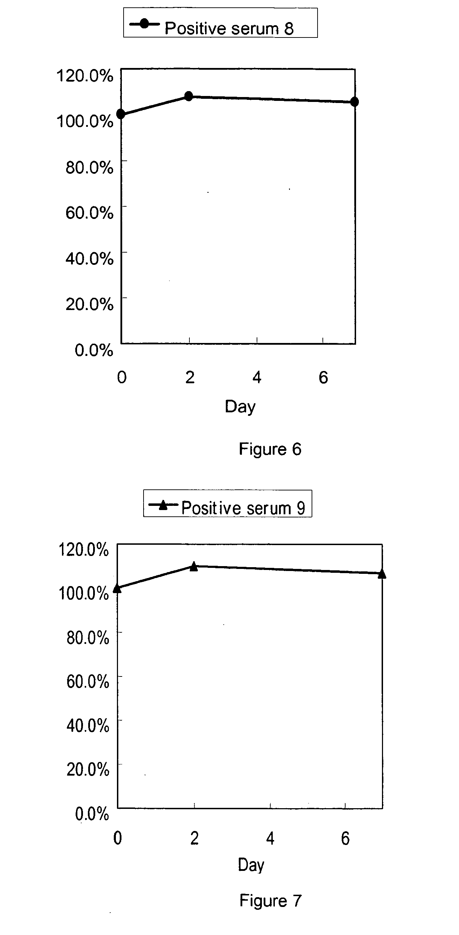

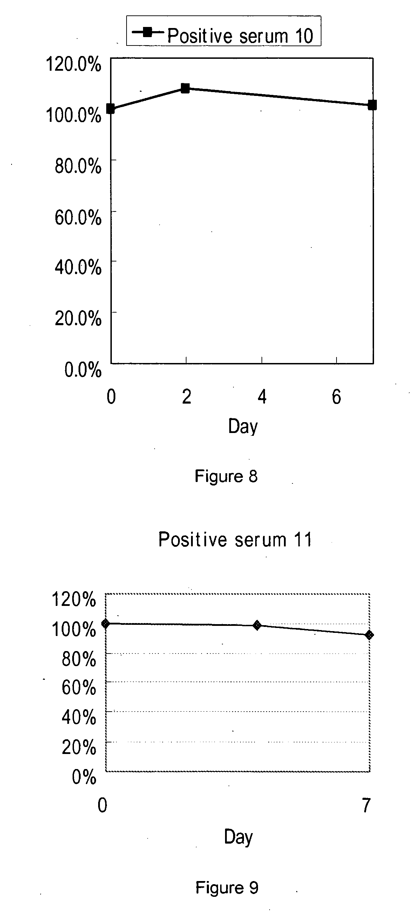

InTable2, the average luminescence intensityobtainedusing the reagents before the acceleration test (day 0) and on days 2 and 7 of the acceleration test is shown. The percentage of average luminescence intensity obtained using the reagents on days 2 and 7 is shown in Figs. 5 to 8 where the average luminescence intensity obtained using the reagents on day 0 for the positive serums 7 to 10 is 100%. Fig. 5 shows the results of measurement of the positive serum 7; Fig. 6 shows the results of measurement of the positive serum 8; Fig. 7 shows the results of measurement of the positive serum 9; and Fig. 8 shows the results of measurement of the positive serum 10. In Figs. 5 to 8, the percentage of average luminescence intensity on each storage duration (days) is shown on the ordinate where the average luminescence intensity obtained using the reagents before the acceleration test (day 0) is 100%. The number of days for which the reagent was stored in the acceleration test is shown on the abscissa.

From the results in Table 2 and Figs. 5 to 8, it was found that a reagent kit comprising the first reagent containing the HCV synthetic peptide antigen in a released state and the second reagent containing the HCV recombinant antigen in a state immobilized on magnetic particles can be used for satisfactorily detecting various samples containing antibodies reacting with various sites of the HCV protein. It was also found that the antigens in the reagent kit are extremely excellent in storage stability.

### Example 3

The first reagent containing the synthetic peptide NS4 antigen and the synthetic peptide core antigen in a released form, and the second reagent containing, in a suspended state, magnetic particles having the recombinant NS3 antigen and streptavidin immobilized thereon, were used in the acceleration test to examine the storage stability of the reagents. In the acceleration test, the first reagent and the second reagent were stored in the dark at 37°C for 0 day, 4 days or 7 days. Each reagent subjected to the acceleration test was used to measure an HCV antibody. In this example, the first reagent (C) used in Example 1 was used as the first reagent.

The magnetic particles having the recombinant NS3 antigen and streptavidin immobilized thereon were prepared in the following manner.

First, commercially available ST magnetic particles were suspended at a density of about 5 mg/mL in 20 mM sodium phosphate buffer, pH 7.5, to prepare an ST magnetic particle suspension. The recombinant NS3 antigen prepared in (1) above was dissolved at a concentration of 1 mg/mL in 20 mM sodium phosphate buffer, pH 7.5, to prepare an NS3 antigen solution. 0.06 mL of the NS3 antigen solution was mixed with 10 mL of the ST magnetic particle suspension and then incubated at 37°C for 30 minutes, whereby the recombinant NS3 antigen was immobilized on the ST magnetic particles. Because the recombinant NS3 antigen used herein was notbiotinylated, the immobilization of the recombinant NS3 antigen onto the ST magnetic particles was due to the physical adsorption thereof onto the magnetic particles. Accordingly, the recombinant NS3 antigen and streptavidin are immobilized respectively on the magnetic particles prepared in this example.

Then, the magnetic particles having the NS3 antigen and streptavidin immobilized thereon were blocked by mixing the magnetic particles with the above-mentioned blocking solution (1% BSA, 20 mM sodium phosphate buffer, pH 7.5) and then incubating the mixture at 37°C for 30 minutes. The magnetic particles thus obtained are hereinafter referred to as the NS3 antigen/ST magnetic particles.

The following second reagent used in this example is designated the second reagent (E).

20 mM sodium phosphate buffer, pH 7.5

5 mg/mL NS3 antigen/ST magnetic particles

The measurement method is as follows:

The measurement was carried out in the same manner as in (15) in Reference Example 1 except that the first reagent (C) subjected to the acceleration test was used as the first reagent, and the second reagent (E) subjected to the acceleration test was used as the second reagent. As the sample, one HCV-negative serum (negative sample 2) and four HCV-positive serums (positive serums 11 to 14) were used. The positive serum 11 is a serum containing an HCV antibody to the NS3 antigen, an HCV antibody to the NS4 antigen and an HCV antibody to the core antigen. The positive serum 12 is a serum containing an HCV antibody to the NS3 antigen. The positive serum 13 is a serum containing an HCV antibody to the core antigen. The positive serum 14 is a serum containing an HCV antibody to the NS3 antigen and an HCV antibody to the NS4 antigen. The results are shown in Table 3.

**Table 3**

| sample | Preservation term | | |
|---|---|---|---|
| | Day 0 | Day 4 | Day 7 |
| | Average luminescence intensity (counts) | Average luminescence intensity (counts) | Average luminescence intensity (counts) |
| Negarive serum 2 | 2.694 | 2.563 | 2.466 |
| Positive serum 11 | 778.724 | 770.584 | 722.425 |
| Positive serum 12 | 599.950 | 557.687 | 541.635 |
| Positive serum 13 | 408.940 | 410.169 | 406.647 |
| Positive serum 14 | 354.613 | 341.189 | 311.943 |

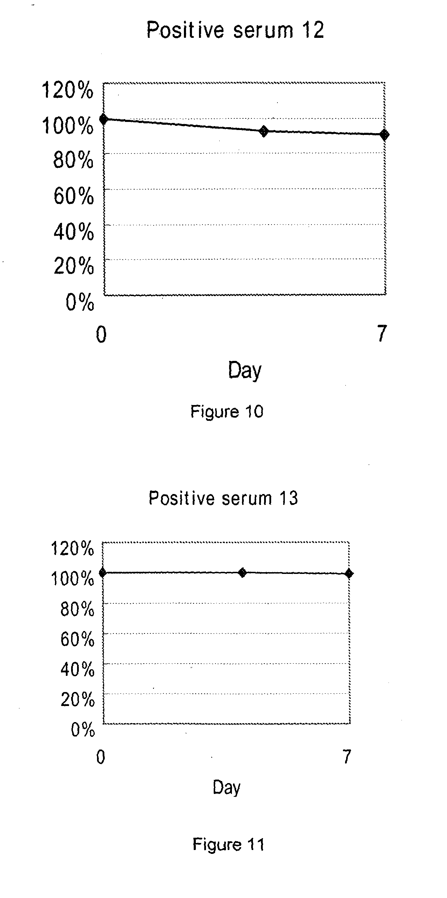

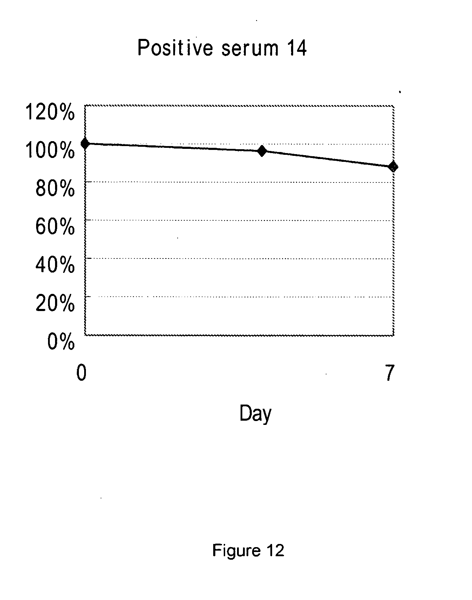

In Table 3, the average luminescence intensityobtainedusing the reagents before the acceleration test (day 0) and on days 4 and 7 of the acceleration test is shown. The percentage of average luminescence intensity obtained using the reagents on days 4 and 7 for the positive serums 11 to 14 is shown in Figs. 9 to 12 where the average luminescence intensity obtained using the reagents on day 0 is 100%. Fig. 9 shows the results of measurement of the positive serum 11. Fig. 10 shows the results of measurement of the positive serum 12. Fig. 11 shows the results of measurement of the positive serum 13. Fig. 12 shows the results of measurement of the positive serum 14. In Figs. 9 to 12, the percentage of average luminescence intensity on each storage duration (days) is shown on the ordinate where the average luminescence intensity obtained using the reagents before the acceleration test (day 0) is 100%, and the number of days for which the reagents were stored in the acceleration test is shown on the abscissa.

From the results in Table 3 and Figs. 9 to 12, it was found that a reagent kit comprising the first reagent containing the HCV synthetic peptide antigen in a released state and the second reagent containing the HCV recombinant antigen in a state immobilized on magnetic particles can be used for satisfactorily detecting various antibodies containing antibodies reacting with various sites of the HCV protein. It was also found that the antigens in the reagent kit are extremely excellent in storage stability.

From the foregoing, it was found the HCV recombinant antigen is more excellent in storage stability and reactivity when immobilized on a solid phase in a buffer, while the HCV synthetic peptide antigen is more excellent in storage stability when not immobilized (in a released state) on a solid phase in a buffer.

## Claims

1. A reagent kit for measuring an HCV antibody comprising:
a first reagent comprising an HCV synthetic peptide antigen which has a solid phase binding site; and
a second reagent comprising a first solid phase which has an HCV recombinant antigen immobilized thereon, and a second solid phase which has a binding substance immobilized thereon,
wherein the binding substance can bind to the solid phase binding site.

2. The reagent kit according to claim 1, wherein a binding substance is also immobilized on the first solidphase, and an HCV recombinant antigen is also immobilized on the second solid phase.

3. The reagent kit according to claim 1 or 2, wherein the HCV synthetic peptide antigen is free in the first reagent.

4. The reagent kit according to any of claims 1 to 3, wherein the HCV recombinant antigen comprises a peptide of non structural protein of HCV.

5. The reagent kit according to any of claims 1 to 3, wherein the HCV recombinant antigen is selected from the group consisting of a recombinant NS3 antigen, a recombinant NS4 antigen, a recombinant NS5 antigen, and a recombinant core antigen.

6. The reagent kit according to any of claims 1 to 5, the HCV synthetic peptide antigen is selected from the group consisting of a synthetic peptide NS4 antigen, a synthetic peptide NS5 antigen, and a synthetic peptide core antigen.

7. The reagent kit according to any of claims 1 to 6, wherein the first reagent comprises a second HCV synthetic peptide antigen different from the HCV synthetic peptide antigen, wherein the second HCV synthetic antigen has a solid phase binding site which can bind to the binding substance.

8. The reagent kit according to claim 7, wherein the HCV synthetic peptide antigen comprises a peptide of structural protein of HCV, and the second HCV synthetic peptide antigen comprises a peptide of non structural protein of HCV.

9. The reagent kit according to claim 7 or 8, wherein the HCV synthetic peptide antigen is a core antigen, the second HCV synthetic peptide antigen is a NS4 antigen or a NS5 antigen, and the HCV recombinant antigen is a NS3 antigen.

10. The reagent kit according to any of claims 1 to 9, wherein the first solid phase and second solid phase are magnetic particles, latex particles or wells of a microtiterplate.

11. The reagent kit according to any of claims 1 to 10, wherein the solid phase binding site comprises biotin, and the binding substance is avidin group.

12. The reagent kit according to any of claims 1 to 11, further comprising a third reagent comprising a substance which can bind to an HCV antibody, and is labeled by a labeling substance

13. The reagent kit according to claim 12, wherein the substance which can bind to a HCV antibody is an antibody.

14. The reagent kit according to claim 12 or 13, wherein the labeling substance is an enzyme, and the reagent kit further comprises a fourth reagent comprising a substrate for the enzyme.

15. A method for measuring an HCV antibody in a sample, comprising:
contacting the sample, a first reagent, and a second reagent,
wherein the first reagent comprises an HCV synthetic peptide antigen which has a solid phase binding site, and the second reagent comprises a first solid phase which has an HCV recombinant antigen immobilized thereon and a second solid phase which has a binding substance immobilized thereon, wherein the binding substance can bind to the solid phase binding site; and
detecting a first complex formed on the second solid phase, and/or a second complex formed on the first solid phase,
wherein the first complex comprises the HCV synthetic peptide antigen and an HCV antibody in the sample, and the second complex comprises the HCV recombinant antigen and an HCV antibody in the sample.

16. The method according to claim 15, wherein a binding substance is also immobilized on the first solidphase, and an HCV recombinant antigen is also immobilized on the second solid phase.

17. The method according to claim 15 or 16, wherein the contacting comprising contacting the sample and the first reagent to prepare a mixture thereof, and contacting the second reagent and the mixture.

18. Use of a first reagent and a second reagent for measuring an HCV antibody in a sample,
wherein the first reagent comprises an HCV synthetic peptide antigen which has a solid phase binding site, and the second reagent comprises a first solid phase which has an HCV recombinant antigen immobilized thereon and a second solid phase which has a binding substance immobilized thereon, wherein the binding substance can bind to the solid phase binding site.

19. The use according to claim 18, wherein a binding substance is also immobilized on the first solid phase, and an HCV recombinant antigen is also immobilized on the second solid phase.

## Patentansprüche

1. Reagenziensatz zum Messen eines HCV-Antikörpers, umfassend:
ein erstes Reagenz, das ein synthetisches HCV-Peptidantigen mit einer Festphasen-Bindungsstelle enthält, und
ein zweites Reagenz, das eine erste Festphase umfasst, die ein daran immobilisiertes rekombinantes HCV-Antigen aufweist, und eine zweite Festphase, die eine daran immobilisierte Bindungssubstanz aufweist, wobei die Bindungssubstanz an die Festphasen-Bindungsstelle binden kann.

2. Reagenziensatz nach Anspruch 1, wobei ebenfalls an der ersten Festphase eine Bindungssubstanz immobilisiert ist, und an der zweiten Festphase ebenfalls ein rekombinantes HCV-Antigen immobilisiert ist.

3. Reagenziensatz nach Anspruch 1 oder 2, wobei das synthetische HCV-Peptidantigen in dem ersten Reagenz frei vorliegt.

4. Reagenziensatz nach einem der Ansprüche 1 bis 3, wobei das rekombinante HCV-Antigen ein Peptid von einem Nichtstrukturprotein des HCV umfasst.

5. Reagenziensatz nach einem der Ansprüche 1 bis 3, wobei das rekombinante HCV-Antigen ausgewählt ist aus der Gruppe, die aus einem rekombinanten NS3-Antigen, einem rekombinanten NS4-Antigen, einem rekombinanten NS5-Antigen und einem rekombinanten Core-Antigen besteht.

6. Reagenziensatz nach einem der Ansprüche 1 bis 5, wobei das synthetische HCV-Peptidantigen ausgewählt ist aus der Gruppe, die aus einem synthetischen NS4-Peptidantigen, einem synthetischen NS5-Peptidantigen und einem synthetischen Core-Peptidantigen besteht.

7. Reagenziensatz nach einem der Ansprüche 1 bis 6, wobei das erste Reagenz ein zweites synthetisches HCV-Peptidantigen enthält, das unterschiedlich zu dem synthetischen HCV-Peptidantigen ist, wobei das zweite synthetische HCV-Peptidantigen eine Festphasen-Bindungsstelle aufweist, die an die Bindungssubstanz binden kann.

8. Reagenziensatz nach Anspruch 7, wobei das synthetische HCV-Peptidantigen ein Peptid des HCV-Strukturproteins umfasst, und das zweite synthetische HCV-Peptidantigen ein Peptid von einem Nichtstrukturprotein des HCV umfasst.

9. Reagenziensatz nach Anspruch 7 oder 8, wobei das synthetische HCV-Peptidantigen ein Core-Antigen ist und das zweite synthetische HCV-Peptidantigen ein NS4-Antigen oder ein NS5-Antigen und das rekombinante HCV-Antigen ein NS3-Antigen ist.

10. Reagenziensatz nach einem der Ansprüche 1 bis 9, wobei die erste Festphase und die zweite Festphase Magnetpartikel, Latexpartikel oder die Vertiefungen einer Mikrotiterplatte sind.

11. Reagenziensatz nach einem der Ansprüche 1 bis 10, wobei die Festphasen-Bindungsstelle Biotin umfasst und die Bindungssubstanz eine Avidingruppe ist.

12. Reagenziensatz nach einem der Ansprüche 1 bis 11, der ferner ein drittes Reagenz umfasst, das eine Substanz enthält, die an den HCV-Antikörper binden kann und die mit einer Markierungssubstanz markiert ist.

13. Reagenziensatz nach Anspruch 12, wobei die Substanz, die an einen HCV-Antikörper binden kann, ein Antikörper ist.

14. Reagenziensatz nach Anspruch 12 oder 13, wobei die Markierungssubstanz ein Enzym ist, und der Reagenziensatz ferner ein viertes Reagenz umfasst, das ein Substrat für das Enzym enthält.

15. Verfahren zum Messen eines HCV-Antikörpers in einer Probe, umfassend:
Inkontaktbringen der Probe mit einem ersten Reagenz und einem zweiten Reagenz, wobei das erste Reagenz ein synthetisches HCV-Peptidantigen umfasst, das eine Festphasen-Bindungsstelle aufweist, und das zweite Reagenz eine erste Festphase enthält, an die ein rekombinantes HCV-Antigen immobilisiert ist, und eine zweite Festphase, an die eine Bindungssubstanz immobilisiert ist, wobei die Bindungssubstanz an die Festphasen-Bindungsstelle binden kann; und
Nachweisen eines ersten Komplexes, gebildet an der zweiten Festphase, und/oder eines zweiten Komplexes, gebildet an der ersten Festphase, wobei der erste Komplex in der Probe das synthetische HCV-Peptidantigen und einen HCV-Antikörper umfasst und der zweite Komplex in der Probe das rekombinante HCV-Antigen und einen HCV-Antikörper umfasst.

16. Verfahren nach Anspruch 15, wobei ebenfalls eine Bindungssubstanz an der ersten Festphase immobilisiert ist und an der zweiten Festphase ebenfalls ein rekombinantes HCV-Antigen immobilisiert ist.

17. Verfahren nach Anspruch 15 oder 16, wobei das Inkontaktbringen das Inkontaktbringen der Probe mit dem ersten Reagenz umfasst, um eine Mischung davon zu bilden, und Inkontaktbringen von dem zweiten Reagenz mit der Mischung.

18. Verwendung von einem ersten Reagenz und einem zweiten Reagenz zur Messung eines HCV-Antikörpers in einer Probe, wobei das erste Reagenz ein synthetisches HCV-Peptidantigen umfasst, das eine Festphasen-Bindungsstelle aufweist, und das zweite Reagenz eine erste Festphase enthält, an die ein rekombinantes HCV-Antigen immobilisiert ist, und eine zweite Festphase, an die eine Bindungssubstanz immobilisiert ist, wobei die Bindungssubstanz an die Festphasen-Bindungsstelle binden kann.

19. Verwendung nach Anspruch 18, wobei ebenfalls eine Bindungssubstanz an der ersten Festphase immobilisiert ist und an der zweiten Festphase ebenfalls ein rekombinantes HCV-Antigen immobilisiert ist.

## Revendications

1. Kit de réactifs destiné à mesurer un anticorps anti-HCV qui comprend :
un premier réactif comprenant un antigène peptidique synthétique du HCV qui a un site de liaison de phase solide ; et
un second réactif comprenant une première phase solide sur laquelle est immobilisé un antigène recombinant du HCV,
et une seconde phase solide sur laquelle est immobilisée une substance de liaison, la substance de liaison pouvant se lier au site de liaison de phase solide.

2. Kit de réactifs selon la revendication 1, dans lequel une substance de liaison est également immobilisée sur la première phase solide, et un antigène recombinant du HCV est également immobilisé sur la seconde phase solide.

3. Kit de réactifs selon la revendication 1 ou 2, dans lequel l'antigène peptidique synthétique du HCV est libre dans le premier réactif.

4. Kit de réactifs selon l'une quelconque des revendications 1 à 3, dans lequel l'antigène recombinant du HCV comprend un peptide d'une protéine non structurale du HCV.

5. Kit de réactifs selon l'une quelconque des revendications 1 à 3, dans lequel l'antigène recombinant du HCV est choisi dans le groupe constitué par un antigène NS3 recombinant, un antigène NS4 recombinant, un antigène NS5 recombinant, et un antigène capsidique recombinant.

6. Kit de réactifs selon l'une quelconque des revendications 1 à 5, dans lequel l'antigène peptidique synthétique du HCV est choisi dans le groupe constitué par un antigène peptidique synthétique NS4, un antigène peptidique synthétique NS5, et un antigène peptidique synthétique de capside.

7. Kit de réactifs selon l'une quelconque des revendications 1 à 6, dans lequel le premier réactif comprend un second antigène peptidique synthétique du HCV différent de l'antigène peptidique synthétique du HCV, le second antigène synthétique du HCV ayant un site de liaison de phase solide qui peut se lier à la substance de liaison.

8. Kit de réactifs selon la revendication 7, dans lequel l'antigène peptidique synthétique du HCV comprend un peptide d'une protéine structurale du HCV, et le second antigène peptidique synthétique du HCV comprend un peptide d'une protéine non structurale du HCV.

9. Kit de réactifs selon la revendication 7 ou 8, dans lequel l'antigène peptidique synthétique du HCV est un antigène capsidique, le second antigène peptidique synthétique du HCV est un antigène NS4 ou un antigène NS5, et l'antigène recombinant du HCV est un antigène NS3.

10. Kit de réactifs selon l'une quelconque des revendications 1 à 9, dans lequel la première phase solide et la seconde phase solide sont des particules magnétiques, des particules de latex ou des puits d'une plaque de microtitration.

11. Kit de réactifs selon l'une quelconque des revendications 1 à 10, dans lequel le site de liaison de phase solide comprend de la biotine, et la substance de liaison est un groupe avidine.

12. Kit de réactifs selon l'une quelconque des revendications 1 à 11, comprenant en outre un troisième réactif comprenant une substance qui peut se lier à un anticorps anti-HCV, et est marquée par une substance de marquage.

13. Kit de réactifs selon la revendication 12, dans lequel la substance qui peut se lier à un anticorps anti-HCV est un anticorps.

14. Kit de réactifs selon la revendication 12 ou 13, dans lequel la substance de marquage est une enzyme, et le kit de réactifs comprend en outre un quatrième réactif comprenant un substrat pour l'enzyme.

15. Procédé de mesure d'un anticorps anti-HCV dans un échantillon, qui comprend :
la mise en contact de l'échantillon, d'un premier réactif et d'un second réactif,
le premier réactif comprenant un antigène peptidique synthétique du HCV qui a un site de liaison de phase solide, et le second réactif comprenant une première phase solide sur laquelle est immobilisé un antigène recombinant du HCV et une seconde phase solide sur laquelle est immobilisée une substance de liaison, la substance de liaison pouvant se lier au site de liaison de phase solide ; et
la détection d'un premier complexe formé sur la seconde phase solide, et/ou d'un second complexe formé sur la première phase solide,
le premier complexe comprenant l'antigène peptidique synthétique du HCV et un anticorps anti-HCV dans l'échantillon, et le second complexe comprenant l'antigène recombinant du HCV et un anticorps anti-HCV dans l'échantillon.

16. Procédé selon la revendication 15, dans lequel une substance de liaison est également immobilisée sur la première phase solide, et un antigène recombinant du HCV est également immobilisé sur la seconde phase solide.

17. Procédé selon la revendication 15 ou 16, dans lequel la mise en contact comprend la mise en contact de l'échantillon et du premier réactif pour préparer un mélange de ceux-ci, et la mise en contact du second réactif et du mélange.

18. Utilisation d'un premier réactif et d'un second réactif pour mesurer un anticorps anti-HCV dans un échantillon, le premier réactif comprenant un antigène peptidique synthétique du HCV qui a un site de liaison de phase solide, et le second réactif comprenant une première phase solide sur laquelle est immobilisé un antigène recombinant du HCV et une seconde phase solide sur laquelle est immobilisée une substance de liaison, la substance de liaison pouvant se lier au site de liaison de phase solide.

19. Utilisation selon la revendication 18, une substance de liaison étant également immobilisée sur la première phase solide et un antigène recombinant du HCV étant également immobilisé sur la seconde phase solide.
